# EUROPEAN PATENT APPLICATION

(11) **EP 1 917 984 A1**
(43) Date of publication of application: **07.05.2008**
(21) Application number: 06022452.4
(22) Date of filing: 27.10.2006
(51) Int. Cl.: A61L 27/32, A61L 27/34

(54) **Coated biomedical device**

(71) Applicant: EMPA Eidgenössische Materialprüfungs- und Forschungsanstalt, CH-8600 Dübendorf (CH)
(72) Inventor: Bruinink, Arie, 8307 Effretikon (CH); Maniura, Katharina, St. Gallen (CH); Kaiser, Jean-Pierre, St. Gallen (CH); Hauert, Roland, 8600 Dübendorf (CH); Balazs, Dawn, St. Gallen (CH); Kern, Philippe, Thun (CH); Schmutz, Patrick, 8600 Dübendorf (CH); Zinn, Manfred, St. Gallen (CH)
(74) Representative: Blum, Rudolf Emil

(57) **Abstract**

The present invention relates coatings for biomedical devices. It further refers to a precursor of such coating intended to be applied to a biomedical device. It further refers to biomedical devices coated with such coatings. It also refers to methods for manufacturing said precursors, coatings and coated biomedical devices as well as a method of applying a biomedical device having a coated surface into a biological environment. The coating comprises a biodegradable polymer and microparticles with a size in the range of 0.5 to 400 μm.

## Description

The present invention relates coatings for biomedical devices. It further refers to a precursor of such coating intended to be applied to a biomedical device. It further refers to biomedical devices coated with such coatings. It also refers to methods for manufacturing said precursors, coatings and coated biomedical devices as well as a method of applying a biomedical device having a coated surface into a biological environment.

Biomedical devices, in particular implants, play an important role in health care today and have an increasing potential for improving both the quality of care and quality of life of the patients and animals. One of the major problems associated with all types of implants is biocompatibility of the implant with the body, and in particular with tissue adjacent to the site of the implant. An absence and/or loss of osteointegration and by that loss of function arises from multiple factors, some of the most important ones are the attachment, proliferation and coverage of the implants by cells not being able to support the function of the implants e.g. in case of artificial joints a coverage by cells not being able to produce bone in order to osteointegrate the implant.

Current implant surfaces are optimized empirically with respect to their in vivo performance. The main motivation for the design of an implant is to take over a specific function which a certain part of the body can no longer fulfil. Secondly, the body reaction should be steered in a way that the implant is accepted or in case of long term implants even is integrated. It is believed that integration of an implant is based on the following sequence of events (phases): 1) attraction of the appropriate cells, 2) proliferation of these in order to obtain coverage of the implant by cells, 3) differentiation of these cells into the for the implant function specific cell type.

It is known that the attraction (phase 1) of appropriate cells can be achieved by release of a chemo attractant, and/or by the presence of adhesion molecules at the implant surface that selectively address membrane proteins of the appropriate cell type and/or reduce the number of other cells that compete with the appropriate cells. Their adhesion should be optimal. However, maximal adhesion counteracts cell migration and proliferation.

Further, it is known that proliferation (phase 2) of and implant coverage by the appropriate cells can be achieved if adhesion of the cells is just large enough for cells to attach and if migration is maximally supported. Furthermore, no queues driving cells into differentiation should be present.

Further, it is known that differentiation of cells (phase 3) is optimal for a cell type or tissue of interest if surface queues are present steering cells into the correct differentiation pathway. It may be assumed that latter is paralleled by a reduced cell migration activity.

A comparison of the optimal surface characteristics of each of these 3 phases (attraction, proliferation, differentiation) makes clear that current surfaces represent a compromise and are far from being optimal for each phase.

Furthermore, surfaces with porosities of 1-0.1 µm may provide niches for bacteria which are not accessible for macrophages. As a result, a chronic inflammation may develop. Although the rate of occurrences of such inflammations is relatively low a surface that would avoid such bacterial hiding just after implantation would represent a clear advantage over currently available structured implants.

The biocompatibility of biomaterials can be determined by several factors depending on the type and status of the biological system as well as on the properties and intended function of the interacting material. The bulk properties of biomaterials are most important for the functionality of the specific device, e.g. the device that depending on its kind must withstand specific load, mechanical wear, aggressive chemical environment, be transparent or electrically conductive, etc. However, as only the material surface is in direct contact with the biological environment, the biological response first of all depends on the material surface properties involved in reactions occurring at the material bioenvironment interface. Therefore, tailoring of biomaterial surface properties for optimal biological response is one of the most important development directions in the biomaterials field.

It is believed that the most important surface properties for material-biosystem interactions are the chemical/physical properties (chemical composition, physical state, surface energy, wettability, hardness, viscoelasticity and surface reactivity) and the topography (e.g. texture, roughness) of the surface. These two classes of properties can be, and often are, correlated to each other, e.g. change in surface topography will affect surface mechanical properties.

GB2397233 discloses a medical device with a coating that is adapted to change with time. This document also addresses the problem of poor integration / acceptance of biomedical devices. However, this coating does not include particles. On the contrary, the aim of the disclosed coating is to provide a surface that is "smoother" when compared with the uncoated implant.

It is an object of the present invention to provide a coating suitable for coating of biomedical devices which overcomes one or more of the aforementioned disadvantages.

It is a further object of the present invention to provide a biomedical device having improved acceptance and/or improved integration properties.

It is a further object of the present invention to provide a biomedical device capable to avoid inflammation reactions.

It is a further object of the invention to provide an improved biomedical device which protects this coating during the implantation.

It is a further aim of the invention to provide processes for manufacturing coatings of (coated) biomedical devices,

In order to address disadvantages of the known biomedical devices, a coating is provided with different surface characteristics over time. Thus, the present invention relates to a biomedical device having a surface with a given topography, said surface is coated with a coating, comprising one or more layers that is adapted to change with time.

Further, a coating is provided which structures are either too small for bacteria to hide or large enough for macrophages to enter enabling to attack all microbes that may adhere to the surface.

Further, the invention relates to an implant having a surface designed to protect parts sensitive to mechanical forces (such as the coatings as described herein) submitted during implantation.

The object of the present invention is achieved by a biomedical device which is adapted to have different surface topographies at different time periods. This has according to the invention been provided by a biomedical device coated with a composition comprising i) biodegradable polymer and ii) microparticles having a size between 0.5-300 µm.

Unless otherwise stated, the following definitions shall apply in this specification:
"Biomedical device" denotes implantable artificial materials and devices in particular i) devices used for clinical applications, such as medical implants (e.g. artificial joints), sensors, instruments, etc., and ii) devices used to handle or modify biological systems, e.g. cell culture substrates, bioreactors, tissue engineering scaffolds, etc. Further examples of biomedical devices are substrates for cell cultures, bioreactors, extra-corporeal life supporting equipment and bioelectronic interfaces. Preference is given to implants. Most biomedical devices have parts made of biomaterials.
"Biomaterials" are nonviable materials used in a device placed in direct contact with biological tissues, organisms or systems, and the main requirement for them is to perform with an appropriate biological response in a specific application, i.e. to be biocompatible.
"Implants" are artificial devices made to replace and act as a missing biological structure, e.g. a hip implant. In this respect it is embraced by the definition of biomedical devices. Implants for human and animal bodies according to this invention are preferably cement-free. Implants may be made of ceramic material, metals (preferably Ti, Cr) or alloys (preferably Ti-alloys, Cr-Mo-alloys), polymeric material (preferably PEEK), reinforced polymeric material (preferably by use of carbon fibers or Carbon nanotubes, or bioglass).
"Coating" refers to a composition that adheres to a biomedical device and covers its surface or parts of its surface.
"Precursor" refers to a composition intended to be applied to a biomedical device to form a coating of said device. Preferably, said precursor is adapted to harden to a solid coating of said biomedical device, said coating is stable in air but degrades, melts and/or resorbs upon contact with a biological environment.
"Biological environment" denotes any environment, where cell tissues may survive such as body tissues or (artificial) body fluids. The term also includes the living human or animal body.

The present invention will be better understood by reference to the figures; a brief description of the figures is given below:
Fig. 1a: implant surface with a coating according to the invention before drying and implantation. (1) implant; (2) (sub) -µm structured surface; with or without antibiotic activity(3) small and large microparticles; (4) degradable polymer; (5) ultrathin layer with or without silver molecules/nanoparticles.
Fig. 1b: the same implant surface as in Fig. 1a but after drying and before implanting.
Fig. 2: Model of an ultrathin protection layer which modifies the degradable polymer surface chemistry and delays polymer degradation. Grey represent functional groups being -COOH or -NH2. An ultrathin protection layer without (A) or with (B) silver molecules/nanoparticulated silver (large black dots) is shown.
Fig. 3 A, B: Schematic representation of a hip implant with square- (A) or oval-like/hexadiagonal (B) indentations. In this embodiment, only the indentations exhibit the coating as described herein.
Fig 3 C: Top view of the implant cut of the level indicated with the arrow on implant B (crosssection), also indicationg the indentations and ridges.
Fig 3 D: Influence of the ridges on bone during implantation. The ridges have two functions. On the one hand it protects the coating according to the invention during hammering the implant into the femur on the other hand it helps to increase the primary stability of the implant directly after implantation.

The present invention will be described in more detail below. It is understood that the various embodiments, preferences and ranges may be combined at will. Preferred are combinations that meet the specific requirements/needs for the biomedical device. Further, depending of the specific embodiment, selected definitions, embodiments or ranges may not apply.

In a first aspect, the invention relates to a coating comprising i) a biodegradable polymer and ii) particles wherein the size of said particles is in the range of 1 to 300 um.

The term Biodegradable polymer refers to a polymer that is adapted to degrade, melt and/or resorb upon contact with a biological environment. Preferably biodegradable polymers fulfil the requirements of EN 13432. Such polymers may be based on either renewable primary products or oil based products. Such polymers are known to the expert in the field. In general, all biodegradable polymers can be used. Such biodegradable polymers are chosen to comply with the material of the biomedical device to be coated, the manufacturing conditions and the intended use of the biomedical device. Typical examples are selected from the group consisting of polyethylene carbonates, polypropylen carbonates, polylactic acids, polyglycolic acids and polyhydroxyalkanoates, e.g. polydroxybutyeate and polyhydroxyvalerate; preferably from the group consisting of polyhydroxyalkanoates and polyethylene carbonates. Cross-linked and non-linked polymers may be used, with a preference to polyethylene carbonates and polyhydroxyalkanoates. For the avoidance of doubt, blends/mixtures of various polymers and blockcopolymers are also within the scope of the invention.

The shrinking properties of biodegradable polymers are an important property of such polymers. According to the present invention, biodegradable polymers may shrink in a range of 10 - 90 % of their original volume, preferably in the range of 30 - 80 % of their original volume. The molecular mass of the biodegradable polymers may vary in a broad range. According to the present invention, biodegradable polymers have a molecular mass in the range of 5.10⁴-5.10⁶ Daltons, preferably in the range of 1.10⁵-1.10⁶ Daltons.

The term microparticle refers to any solid material that is compatible with the biological environment and has a size of 1 - 300 µm. Such particles are known. Typical examples are selected from the group consisting of calciumphosphates, bioglass, polylactic acids, polyhydroxyalkanoates and polyglycolic acids, also including mixtures thereof such as mixtures of different calciumphosphates and mixtures containing polylactic acids and/or polyhydroxyalkanoates and/or polyglycolic acids.

The shape of the microparticles may vary including irregularly (square) shaped microparticles and regularly shaped microparticles. A preference is given to spherical or globular shaped microparticles.

The size of the microparticles may vary according to the invention in the range of 1 - 300 µm. Size ranges are defined as the interval wherein 90 % of the microparticles are within the given range and may be determined by SEM.

In a preferred embodiment, the microparticles have a bimodal size distribution. That is one group of microparticles is "small", while the other group is "large". Small microparticles may be in the range of 1 - 10 µm, e.g. 5 µm; large microparticles may be in the range of 15 - 45 µm, e.g. 30 um. The ratio of "small" to "large" microparticles may vary in a broad range, in general between 20 - 80 wt-% small microparticles (and correspondingly 80-20 wt-% large microparticles), preferably 40 - 60 % small microparticles, e.g. 50% small microparticles.

The microparticles as described herein represent the structuring entities of the coating. It is believed that said microparticles can promote cell proliferation and migration.

The coating may have a thickness in a broad range, according to the need of the end-user, but typically in the range of 20 - 200 µm when implanted, preferably 40 - 100 µm.

In a preferred embodiment, the microparticles are made of biodegradable material and are stable for at least 5 days when implanted and are fully degraded within 2 years when implanted.

In a preferred embodiment, the biodegradable polymer is selected from the group of polyhydroxyalkanoates polymers and the microparticle is selected from the group of calciumphosphates.

In another preferred embodiment, the biodegradable polymer is selected from the group of polyethylene carbonates polymers and the microparticle is selected from the group of calciumphosphates.

In a further preferred embodiment, the biodegradable polymer and the microparticles are selected from the same group of compounds, e.g. both components are of polylactic acids or polyhydroxyalkanoates or polyglycolic acids.

In a further embodiment, the coating as described above is complemented by an additional ultra thin protection layer, covering the first coating layer. Such an ultrathin layer is provided on top of the previous coating. It is not structured by its own but completely follows the structure of the underlying coating. This layer has the function to retard degradation of the underlying (first) layer. In an advantageous embodiment, the ultrathin layer may comprise an effective amount of one or more compounds having antibacterial properties. Suitable polymers for use in connection with the ultrathin layer are stable for at least 5 days when implanted and are fully degraded within 15 days when implanted. Such polymers are e.g. acrylic acid based polymers with low carbonyl group density. The ultra thin layer may be applied e.g. by using plasma coating and/or plasma treatment techniques. The ultra thin layer may have a thickness of 0,1 - 100 nm, preferably 10 - 50 nm.

In an advantageous embodiment, the ultrathin protecting layer contains compounds that have antibiotic properties, such as commercially available organic compounds and metals, such as silver. A preferred compound that has antimicrobiotic properties is silver. Silver may be present in the ultrathin protecting layer in an effective amount in the form of nanoparticles. The term nanoparticle refers to any solid material that is compatible with the biological environment and has a size of 1 - 100 nm. Such nanoparticles are known.

Depending on the various embodiments of the present invention, the materials disclosed may be provided to the end-user in different ways. If it is intended to use a coating without an ultrathin protecting layer, it can be delivered (i) as a part of the biomedical device and thus be applied to the device in the production plant or (ii) as a separate product for application to an existing commercial biomedical device in clinics or laboratories. In the latter case (ii), the coating is delivered as a precursor, which may be deposited directly on the biomedical device surface or be melted or dissolved to a fluid phase before deposition on the implant surface. If, on the other hand, it is intended to use a coating with an ultrathin protecting layer, it is preferably delivered as a part of the biomedical device and thus be applied to the device in the production plant.

When brought in contact with a biological environment, such as body tissue or body fluid, the ultrathin protection layer and microparticle containing coating layer should melt or resorb/degrade in a timely manner (as described herein) into biocompatible products, which can be digested by the body without inducing adverse effects.

In a second aspect, the invention relates to a biomedical device coated with a coating as described herein.

The term biomedical device is described above. In a preferred embodiment, the biomedical device is an implant. Any suitable material may be used for such an implant. Preferably, such implants are made of biomedical grade steel, Ti, Ti-alloys, Cr, Cr-Mo alloys, and Co-alloys.

In a further embodiment, the surface of the biomedical device may be treated with bacteriostatic / toxic molecules e.g. furanones (as described in Baveja et al., (2004) Biomaterials 25, 5003-12) or polypeptides with an amino sequence including KPV (as described in Cutuli et al., (2000) J. Leukoc. Biol. 67, 233-9)). Such treatment of the surface has a beneficial influence, as it is believed that it inhibits bacterial growth.

For biomedical devices other than implants, it could also be desirable to have different surface topographies at different time periods. One example of such a biomedical device is a tissue engineering scaffold, which is used in vitro or in vivo to generate tissues or organs from a group of cells growing on some form of porous, three- dimensional shaped substrate ("scaffold"). Other similar examples are substrates for cell cultures, bioreactors, extra-corporeal life supporting equipment and bioelectronic interfaces. For example, it may be beneficial to use one kind of topography to induce higher motility of specific cell types so that those cells can spread over the substrate and follow guiding topographic cues into specific locations. Later, another type of topography would be desirable for switching-on proliferation cycle in the cells, and finally, determine cell differentiation.

In a further preferred embodiment, the implant is equipped with cavities (also referred to as indentations) on the surface of said implant. Such cavities are known and described e.g. in WO03/061516, WO03/053669, EP033011, US4530116. The shape of these cavities is not restricted to a particular one. Cavities, with or without an undercut, with a planar or otherwise formed surface, are within the scope of the invention. Typically, cavities have a depth of 0.5-3 mm and an area of 0.5-2 cm². The cavities may be arranged in a geometric pattern. Further, only a part of the implant may be equipped with said cavities. The coating as described herein may be applied only to the cavities or to the whole implant surface. Preferably, only the cavities are coated.

In case the implant is equipped with cavities, the remaining surface may be treated in a way to obtain a smooth surface, e.g. by polishing said remaining surface prior to or after the coating.

In a further preferred embodiment, the biomedical device has a (sub)-micrometer structured surface preferably at that part of the surface that is coated. As the term implies this refers to a roughness of the surface which is below the micrometer scale. Such surfaces may be obtained by known methods, i.e. by sand blasting, anodizing or by etching techniques. The thickness of the (sub)-micrometer structured surface is in the range of 0,04 - 100 µm.

In a further aspect, the invention relates to a coating precursor comprising i) a biodegradable polymer as described herein ii) microparticles as described herein and iii) one or more solvent(s).

Suitable solvents are able to dissolve the polymer used and to disperse (but not dissolve) the microparticles used. It is within the ordinary skill to select solvent(s) appropriate for the polymer and microparticle used. For example, water is a suitable solvent for the combination of a polyhydroxyalkanoate or polyethylene carbonate and calciumphosphate. For example, water : methylenechloride = 2: 1 is a suitable solvent for the combination of polyhydroxyalkanoate and calciumphosphate.

Further, the coating precursor should wet the biomedical device surfaces and have an adjustable viscosity.

After deposition, which may take place through dipping of the device in the precursor phase, the coating precursor should harden. The hardening can take place for example by curing, drying, solvent removal or freezing preferably by solvent removing.

In a further aspect, the invention relates to a process for manufacturing a coating precursor comprising the step of mixing one or more solvent(s), a biodegradable polymer and microparticles as described herein.

In a further aspect, the invention relates to processes for manufacturing a biomedical device as described herein.

In one embodiment, the process for manufacturing a biomedical device as described herein comprises the step of i) optionally establishing a submicrometer structured surface on the implant (e.g. by sand blasting or etching techniques); ii) coating the biomedical device with a coating precursor (e.g. by dip coating, spray-coating or comparable technique); iii) removing the solvent from the coated device (e.g. by exposing to solvent saturated air and/or applying reduced pressure) and iv) optionally applying an ultrathin protecting layer to the coated device (e.g. by plasma coating).

In a further embodiment the process for manufacturing an implant comprises the step of i) dip-coating or spray-coating an implant with a coating precursor as described herein and ii) removing the solvent from the coated implant.

In a further embodiment, said process comprises the step of i) dip-coating or spray-coating a biomedical device with a coating precursor according to claim 13 ii) removing the solvent from the coated device and iii) applying an ultrathin protecting layer to the coated device.

According to a further aspect, the invention relates to a process of implanting a biomedical device comprising the step of applying a coated biomedical device as described herein into a biological environment.

According to a further aspect, the invention relates to the use of a biomedical device as described herein for implanting in a biological environment.

Without being bound to theory, it is believed that a suitable selection of the components of the coating as described herein provides a method for controlling tissue - implant interactions in a time dependent way. This allows better integration, function and extended lifespan of implants in the body.

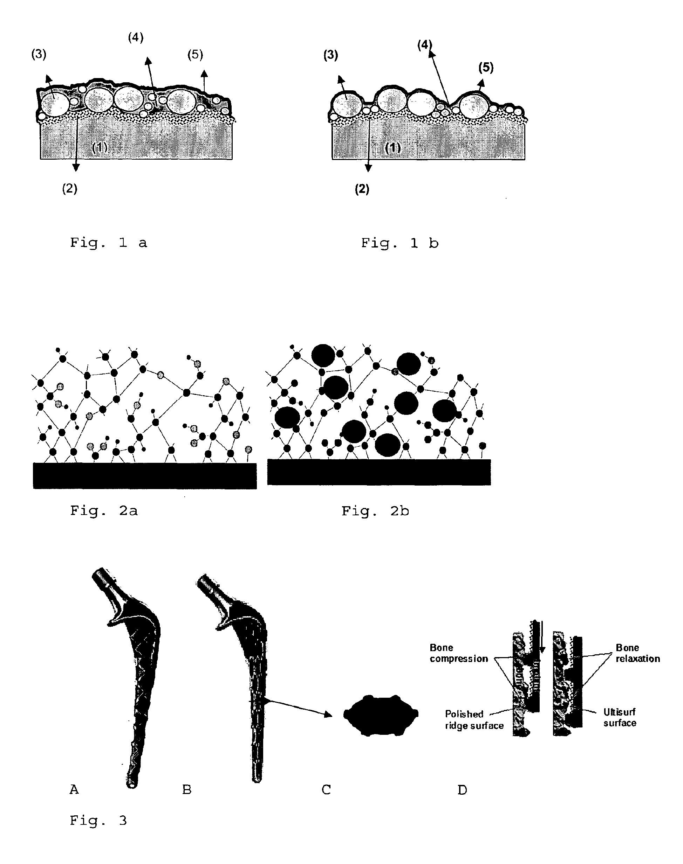

Without being bound to theory, it is believed that the beneficial properties of the coatings and devices as described herein are based on the following considerations: An implant, which optionally is equipped with (sub-)micrometer structured surface and /or antibiotic molecules, is coated by spraying, dip-coating or other comparable technique with a degradable polymer. Latter polymer contains microparticles with typical sizes of 30 and 5 µm. As a result of the removal of the solvent and shrinking of the polymer a surface is created with half-sphere-like structures with a diameter of around 30 µm (Fig. 1). It is assumed that such surfaces stimulate cell proliferation and migration. Further, the surface that becomes accessible for cells after degradation of the top layers is stable and exhibits structures of the submicrometer range. It is believed that latter surfaces stimulate osteointegration and represent the current common surface of commercially available implants (e.g. hip prostheses). Preferably, the intended effect of the microparticle-polymer composite coating may further be supported by an ultrathin layer (1-100 nm) to enhance adhesion of cell of interest and to support their proliferation. Said ultrathin protection layer should have properties to delay the degradation of the underlying layer for approximately 5 days. Latter protection layer may also contain compounds as separate molecules or as nanoparticles (preferably silver) that are known to be antibiotic in order to prevent biofilm formation at the implant surface.

Without being bound to theory, it is believed that the obtained surface will not be fully resistant against large physical stress. In order to protect the coatings during the implantation procedure the implant is designed in a way that the implant surface consists of (0.5-2cm)X(0.5-2 cm) and around 0.5-3 mm deep indentations (Fig. 3). In a preferred embodiment, only the surface of such indentations will exhibit the coating as described (Fig. 3). The top of the ridges may be polished to help the sliding into the bone during implantation and since a structured surface may not withstand the local physical forces giving result to metal particles. Latter could have an adverse effect on contacting cells. Since only the ridges directly contact the bone during the implantation process such overall design helps to protect the coating as described herein. Furthermore, on the moment that the implant is hammered into the bone for instance the femur the bone is locally compressed where the implant ridges come in contact with the bone (Fig. 3C left). At each stroke with the hammer the ridges are dislocated (Fig. 3C right) and the bone will relaxate at areas where the indentations are located since bone is elastic to a certain extend (and of course partly will be scraped into the cavities). Latter gives the patient important primary stability of the implant within the bone.

Without being bound to theory, it is believed that the coating (even in absence of antimicrobiotic compounds such as silver) additionally hinders bacteria to penetrate and bed into the stable submicrometer structured implant surface (promoting cell differentiation) that become accessible to cells after degradation of both coatings, thus avoiding inflammation reactions. The protection for the bacterial hiding inside the submicrometer structure will by that be present as long as the coating layer covers the (sub-)micrometer structured surface, i.e. during implantation and the first period thereafter. After the coating is degraded the final implant surface contacts the biological environment. In order to prevent microbial colonization of the implant surface as result of a hematogenic infection the final implant surface may bare antibiotic molecules.

In summary, an implant as described herein may have one or more of the following important advantages:
- Induced faster tissue regeneration around the implant, due to a reduced destruction of contacting tissue.
- Better stability of original implant surface during the storage, transportation and insertion periods.
- Improved implant integration and thus lower long-term failure rate.
- Faster healing and patient treatment.
- Reduced initial inflammatory reaction, which may be caused when inserting an implant having a rough surface in which bacteria can hide.
- In case silver is included in the ultrathin protection layer an additional insurance that bacteria are killed in case that they are attached to the pristine coated surface of the implant directly after implantation.

The invention will now be described by way of example. These examples are meant to illustrate the invention, with no intend to a limitation.

Example 1: Manufacture of coating precursor: 1 g of Poly-(Hydroxybutyrate) are added to 20 g of methylene chloride and stirred until a clear solution is obtained. 1 g of calcium-phosphate microparticles with sizes of 5 and 30 µm (ratio 5:1) are added and mixed again for 5 h to obtain a dispersion.

Example 2: Coating of an implant: The shaft of a commercially available hip implant is coated with the dispersion of example 1 by spraying 0.8 g of the dispersion using air brush device. The coated implant is dried for 48 h at 60 °C in dry air. The obtained coated implant has a coating thickness of 35-40 µm, as determined by profilometry.

Example 4: improved acceptance of coated implant: The improved acceptance of the implants according to the invention is shown by the following test: cultivation of human bone cells for 7-14 days on top of the samples with the various coatings under proliferation and osteogenic medium conditions. Cell proliferation and cell differentiation are measured according standard procedures.

## Claims

1. A coating comprising i) a biodegradable polymer and ii) microparticles wherein the size of said micro-articles is in the range of 0.5 to 400 µm.

2. A coating according to claim 1 wherein said microparticles have a bimodal size distribution.

3. A coating according to claim 1 or 2, wherein the biodegradable polymer is selected from the group consisting polyhydroxyalkanoates and polyethylene carbonates.

4. A coating according to any of claims 1 to 3, wherein the microparticles are selected from the group consisting of calciumphosphates.

5. A biomedical device coated with a coating according to any of claims 1 to 4.

6. A biomedical device partly coated with a coating according to any of claims 1 to 4.

7. A biomedical device according to claim 6, wherein the coating is present in cavities of said device.

8. A biomedical device according to claim 6 or 7, wherein the uncoated part of the surface is polished.

9. A biomedical device having a (sub-)micrometer-structured surface and coated with a coating according to any of claims 1 to 4

10. A biomedical device having a (sub-)micrometer-structured surface and partly coated with a coating according to any of claims 1 to 4

11. A biomedical device according to claim 10, wherein the coating is present in cavities of said device.

12. A biomedical device according to claim 10 or 11, wherein the uncoated part of the surface is polished.

13. A biomedical device according to any of claims 5 to 12 further comprising an ultrathin protecting layer.

14. A biomedical device according to claim 13 wherein the ultrathin protecting layer is selected from the group consisting of acrylic acid based polymers.

15. A biomedical device according to claim 13 wherein the ultrathin protecting layer contains an effective amount of one or more compounds having antibiotic activity.

16. A biomedical device according to claim 15 wherein the compounds having antibiotic activity is silver, preferably in the form of nanoparticles.

17. A biomedical device according to any of claims 5-16 wherein said device is an implant.

18. A coating precursor comprising a coating according to any of claims 1 to 4 and one or more solvents, wherein the solvent(s) can disperse or dissolve the biodegradable polymer.

19. A process for manufacturing a biomedical device according to any of claims 5 to 17 comprising the step of i) dip-coating or spray-coating a biomedical device with a coating precursor according to claim 16 and ii) removing the solvent from the coated device.

20. A process for manufacturing a biomedical device according to claims 13 or 16 comprising the step of i) dip-coating or spray-coating a biomedical device with a coating precursor according to claim 18, ii) removing the solvent from the coated device and iii) applying an ultrathin protecting layer to the coated device.

21. A process for manufacturing a coating precursor comprising the step of mixing a solvent, a biodegradable polymer and microparticles.

22. A process of implanting a biomedical device comprising the step of applying a device according to any of claims 5 - 17 into an artificial biological environment.

23. Use of a coating according to any of claim 1 - 4 for impregnating a biomedical device.

24. Use of a biomedical device according to any of claims 5 - 12 for use in an artificial biological environment.
